# EUROPEAN PATENT SPECIFICATION

(11) **EP 3 620 137 B1**
(45) Date of publication and mention of the grant of the patent: **27.11.2024**
(21) Application number: 19195838.8
(22) Date of filing: 06.09.2019
(51) Int. Cl.: A61F 5/455

(54) **MENSTRUAL CUP**
MENSTRUATIONSBECHER
COUPELLE MENSTRUELLE

(30) Priority: 07.09.2018 US 201816124262; 27.09.2018 US 201816143794
(43) Date of publication of application: 11.03.2020
(73) Proprietor: The Procter & Gamble Company, Cincinnati, OH 45202 (US)
(72) Inventor: Stoebe-Latham, Rebecca, Cincinnati, Ohio 45202 (US)
(74) Representative: P&G Patent Germany

(56) References cited:
- WO-A1-2007/082341
- US-A1- 2003 023 223
- US-A1- 2015 351 975
- US-A1- 2017 049 609
- ROSEY REUSABLES: "JuJu Cup Model 4 - Menstrual Cup Review", YOUTUBE, 20 February 2018 (2018-02-20), pages 1 - 3, XP054980135, Retrieved from the Internet <URL:https://www.youtube.com/watch?v=is3HlaxN-64> [retrieved on 20200122]
- ROSEY REUSABLES: "JuJu Cup Model 4 vs. Super Jennie Small (Menstrual Cup Comparison)", YOUTUBE, 1 February 2018 (2018-02-01), pages 1 - 1, XP054980134, Retrieved from the Internet <URL:https://www.youtube.com/watch?v=6gIcY2Gf7qM> [retrieved on 20200122]
- ANONYMOUS: "Choosing the right size menstrual cup. What model JuJu cup do I need?", 22 January 2020 (2020-01-22), XP055660809, Retrieved from the Internet <URL:https://www.juju.com.au/menstrual-cup-size-guide/> [retrieved on 20200122]

## Description

### FIELD OF THE INVENTION

The present disclosure generally relates to a menstrual cup having specific geometric properties to optimize comfort and protection. The disclosure further relates to an array of packages comprising feminine consumer articles and kits comprising a variety of feminine consumer articles.

### BACKGROUND OF THE INVENTION

Menstrual cups, and other vaginal discharge collection devices are well known. The first menstrual cups were designed in 1930 but have only become more popular commercially over the past 10 years. While user acceptance of such devices is increasing, there are still many issues with existing menstrual cups. Menstrual cups are intended to be re-used, requiring changing and cleaning once every 8 to 12 hours, dependent on flow. Menstrual cups also need to be comfortable, they need to maintain their position within a user's body and they need to be easy to insert and remove. This results in multiple natural tradeoffs when designing menstrual cups.

For example, if the shape of a menstrual cup is rounder, it will generally be easier to insert and remove, but may push more on the bladder resulting in discomfort for a user. Alternatively, cups that are very stiff tend to open and seal to vaginal walls easily, but stiff cups can conform to the body less and may be less comfortable or may shift more during use. Thus, there exists some natural tradeoffs between, for example, capacity of the cup and comfort or flexibility of the cup and rigour.

Cups that are currently on the market have differing design choices that lead to shortcomings in the product design. For example, one cup has a very round base, which increases overall capacity of the cup, but that pushes against the bladder and may cause difficulty with urination. Another available cup is long and formed of more flexible material, which can cause discomfort and may not open as easily as other cups. There are various such problems with cups present on the market today.

Thus, there exists a need for a cup that solves one or more of the above identified problems.

### SUMMARY OF THE INVENTION

The invention is defined by the claims.

### BRIEF DESCRIPTION OF THE DRAWINGS

FIG. 1 is a perspective view of one example of a menstrual cup as described herein.
FIG. 2 is a front view of the menstrual cup in an upright position, indicating the vertical and horizontal axes.
FIGs. 3A and 3B show different dimensions of the cup when viewed through a cross-section.
FIG. 4 shows dimensions of the wall of the menstrual cup when viewed through cross-section x-x.

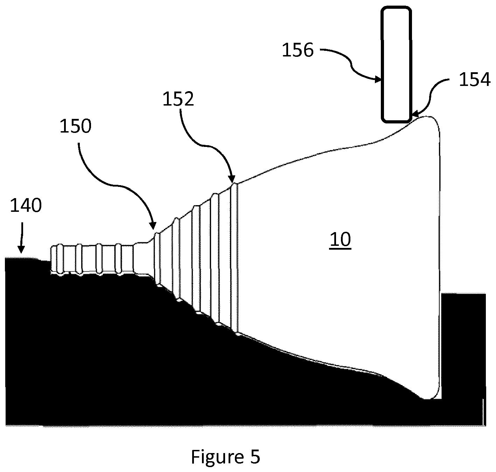

FIG. 5 schematically illustrates the process of taking measurements according to the compression test method.
FIGs. 6 and 7 show in graph format, the relative fit of prior art menstrual cups.
FIG. 8 shows in graph format the relative fit of the menstrual cup described herein.

While the specification concludes with claims particularly pointing out and distinctly claiming the subject matter that is regarded as the present invention, it is believed that the invention will be more fully understood from the following description taken in conjunction with the accompanying drawings. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. The drawings are not necessarily to scale.

### DETAILED DESCRIPTION

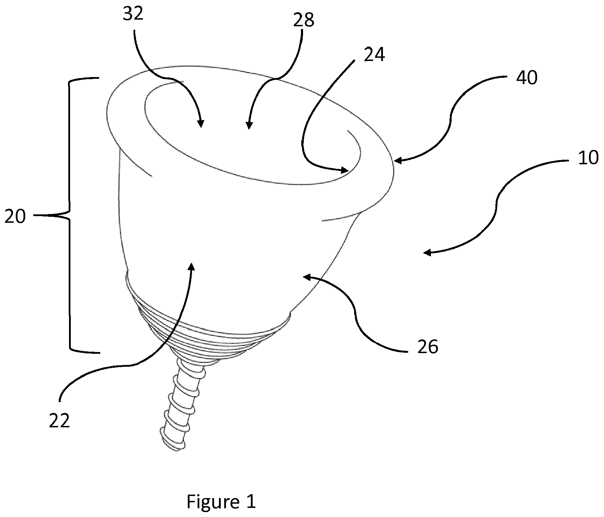

Reference is made herein to a menstrual cup 10, as shown in Figure 1. However, it will be appreciated that the device described may be used for collection of other vaginal fluids. To insert the menstrual cup in a user's vagina, an open end of the cup must first be folded in on itself and held in folded position by a user. Once located in position, the user releases the cup, at which point it unfolds and nestles within the user's vagina. Thus, the cup needs to be sufficiently flexible to enable folding ready for insertion, but it should also have enough resilience to resume its original position once released. To remain in position, the cup must have a certain width such that it remains "wedged" around the cervical OS, without being so wide that it causes discomfort for a user. It is known that when menstrual cups are too big, they may cause discomfort and/or e.g., press on the bladder of a user. To remove the cup, a user grasps the base of the cup or, where applicable, a stem provided at the base of the cup and slowly pulls the cup from the vagina. During this process, the open end of the cup squeezes together, temporarily reducing the capacity of the cup. If there is too much liquid within the cup during removal, there may be some spillage. Conversely, for optimal usage time without having to replace and/or clean the cup, the cup should have a certain capacity in line with the flow of average users. Thus, there are many trade-offs that must be made and factors that should be balanced.

Furthermore, for optimal usage, it may be necessary to provide an array of cups that cater for different needs, e.g. vagina size and fluid flow. The present inventors have discovered that the needs of most users can be met with appropriately chosen dimensions of two or more cups as described herein.

### Receptacle

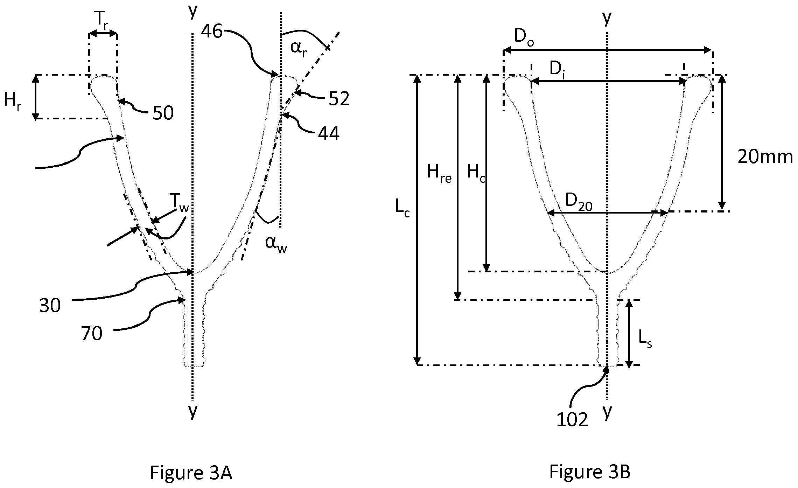

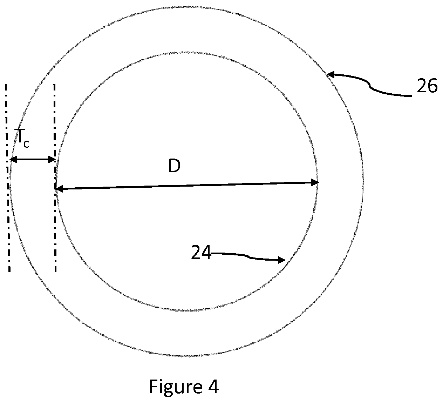

The cup 10, which is adapted for use in a vagina, includes a receptacle 20 having a wall 22 with an inner wall surface 24 which defines a cavity 32 adapted for collecting fluid and an opposed outer wall surface 26. The receptacle extends from an open top end 28 to a closed bottom end 30 (shown in Figure 3A). When viewed through the y-y cross-section (as shown in Figures 3A & 3B), the receptacle has a generally "V" shaped cross-section. When viewed through the x-x cross-section (as shown in Figure 4), the receptacle has a generally cylindrical cross-section that increases in diameter from the base of the receptacle to the open top end.

The wall includes an upper rim portion 40 which strengthens the top end of the receptacle, maintaining the cup in a pre-selected position when in situ and providing appropriate resiliency for the cup to resume its shape once in situ. The wall further includes a main portion 42 extending from the bottom end towards the top end. The wall additionally includes a transition portion 44 between the main portion and the rim portion.

The thickness of the cup wall, measured between the inner wall surface 24 and the outer wall surface 26 varies from the rim portion 40 to the main portion 42, as best seen in Figure 3A & 3B. Generally, the thickness of the wall decreases from the rim portion 40 to the transition portion 44 and from the transition portion 44 to the main portion 42. The rim portion of the wall is rounded at its top end 46 and forms the thickest part of the wall, thus strengthening the top end and maintaining both cup shape and position when in situ. The transition portion 44 of the wall tapers from the thicker rim portion to the main portion which is narrower. The transition portion permits the cup to maintain a substantially smooth profile for increased comfort during use due.

Preferably, the thickness Tᵣ of the rim portion of the wall, as measured at the thickest part of the rim in a direction substantially orthogonal to the vertical axis of the cup, is between 3.5 mm and 5.5 mm, 4 mm and 5 mm, 4.25 mm and 4.75 mm measured between the inner surface 50 and the outer surface 52 of the rim. A ratio of Tᵣ to T_{w} is preferably at least 1.1:1, 1.2: 1, 1.25:1, 1.3:1, 1.4:1 or 1.5:1.

The rim further has a height Hᵣ measured in a direction substantially parallel to the longitudinal axis from the open top end of the receptacle to the transition portion, where the thickness of the wall reduces. Preferably, the height Hᵣ is between 5 mm and 15 mm, 6 mm and 13 mm, 7 mm and 11 mm or 8mm and 9mm.

The outer surface 52 of the rim portion of the wall further has an angle αᵣ relative to the longitudinal axis that is greater than the angle α_{w} of the outer surface of the main portion of wall relative to the longitudinal axis. The point at which the angle changes is also at the transition portion 44 of the wall. Without being bound by theory, it is thought that this increased angle provides better anchorage for the cup when in situ. Preferably, the angle of the outer surface of the upper rim portion relative to the longitudinal axis is between 28° and 34°, 29° and 33°, 30° and 32° or 31°.

The rim preferably has a rounded profile to improve comfort of the cup when in situ.

The thickness of the wall T_{w} remains generally constant through the main portion, although it will be appreciated that this thickness could vary, for example, becoming narrower towards the open top end (to the rim). The narrower wall thickness in the main portion allows ready folding of the cup for both insertion into and removal from the vagina. Preferably, the main portion of the wall has a thickness T_{w} of between 1 mm and 5 mm, 1.5 mm and 4.5 mm, 2 mm and 4 mm, 2.5 mm and 3.5 mm or 3 mm.

At the transition portion, there is preferably a radius of curvature between the wall at the rim and the wall at the main portion of between 6 mm and 9 mm or 7 mm and 8 mm. The radius of curvature ensures a smooth transition from the rim to the main portion of the wall, thereby ensuring a comfortable fit while the cup is in situ and smooth entry and removal of the cup from the user's vagina.

As the dimensions described above (wall thickness at different points, angle of outer surface of rim relative to longitudinal axis and height of rim) are chosen for their properties in terms of shape maintenance, foldability and resilience, it is anticipated that they may be kept the same throughout an array of cups of different sizes. Thus, preferably, the first and second cups of an array of cups have substantially the same wall thickness (Tᵣ and T_{w}), Rim angle (αᵣ), rim height (Hᵣ) and radius of curvature.

As mentioned briefly above, the receptacle has a general "V-shaped" cross-section. Compared with existing menstrual cups, it has been found that the "V-shaped" cross-section provides an optimal shape that balances the need for a wide open end to hold the cup in place when in situ, with a narrower lower end to avoid discomfort e.g., caused by the cup pushing on the bladder, while maximising the overall capacity of the cup.

Considering first the outer surface of the wall, the receptacle has a diameter Dₒ measured at the open top end measured between the widest points of the cup at the upper rim portion of the wall in a direction substantially orthogonal to the longitudinal axis. A second diameter D₂₀ is measured between the outer surface of the wall at a point 20 mm away from the open top end of the receptacle (in a direction parallel substantially parallel to the longitudinal axis). The relationship between these diameters provides an indication of the overall structure of the cup. Preferably, the ratio of D₂₀ to Dₒ should be less than 0.9, preferably less than 0.85, preferably less than 0.8.

Preferably Dₒ is between 40 mm and 55 mm, 42 mm and 53 mm, 45 mm and 49 mm, 46 mm and 48 mm, 46 mm and 54 mm, 47 mm and 53 mm or 52 mm and 54 mm. In an array of cups of different sizes, preferably a first cup has a diameter Do of between 45 mm and 49 mm, 46 mm and 48 mm or 47 mm and a second cup has a diameter Do of between 51 mm and 55 mm, 52 mm and 54 mm or 53 mm. It is anticipated that this range of rim diameters in an array of cups would fit the 86 percentile of potential users.

The receptacle further has a height Hᵣₑ measured from the open top end of the receptacle to an external base 70 in a direction substantially parallel to the longitudinal axis. Preferably, the height Hᵣₑ is between 43 mm and 50 mm, 44 mm and 49 mm, 45 mm and 48 mm, 46 mm and 47 mm. Where different sizes of cups are provided, a first cup may have a height Hᵣₑ of between 43 mm and 49 mm, 44 mm and 48 mm, 45 mm and 47 mm or 46 mm and the second cup may have a height Hᵣₑ or between 46 mm and 51 mm, 47 mm and 50 mm, 48 mm and 49 mm.

The capacity of the cup is determined by the inner dimensions of the receptacle, for example, the inner diameter, Dᵢ, measured at the open top end between inner surfaces of the wall, and the cavity depth H_{c} measured from the open top end to the cavity base. Furthermore, the capacity of the cup varies when in use from a "resting position" i.e., when in situ in a user's vagina or when outside of the body or in "compressed" position, i.e., during insertion or removal from the user's vagina. The difference between the compressed capacity and the resting capacity should not be so extreme that any liquids held in the cup are spilled during removal of the cup. Thus, in embodiments, the receptacle or cup has a resting capacity Cᵣ of between 20 mL and 40 mL, 22 mL and 38 mL. As described above, where an array of cups is provided, preferably a first cup has a capacity of between 20 mL and 28 mL, 21 mL and 27 mL, 22 mL and 26 mL, 23 mL and 25 mL or 24 mL and a second cup has a capacity of between 33 mL and 41 mL, 34 mL and 40 mL, 35 mL and 39 mL, 36 mL and 38 mL or 37 mL.

The depth of the cavity as measured between the open top end and the cavity base in a direction substantially parallel to the longitudinal axis is between between 40 mm and 47 mm, 41 mm and 46 mm, 42 mm and 45 mm, 43 mm and 44 mm. Where different sizes of cups are provided, a first cup may have a cavity depth H_{c} of between 40 mm and 46 mm, 41 mm and 45 mm, 42 mm and 44 mm or 43 mm and the second cup may have a cavity height H_{c} of between 46 mm and 51 mm, 47 mm and 50 mm, 48 mm and 49 mm.

The diameter of the cavity Dᵢ as measured between the inner surfaces of the wall at the open top end may be between 34 mm and 48 mm, 36 mm and 46 mm, 38 mm and 44 mm, 40 mm and 42 mm. In an array of cups of different sizes, preferably a first cup has a diameter Dᵢ of between 36 mm and 40 mm and a second cup has a diameter Dᵢ of between 42 mm and 46 mm.

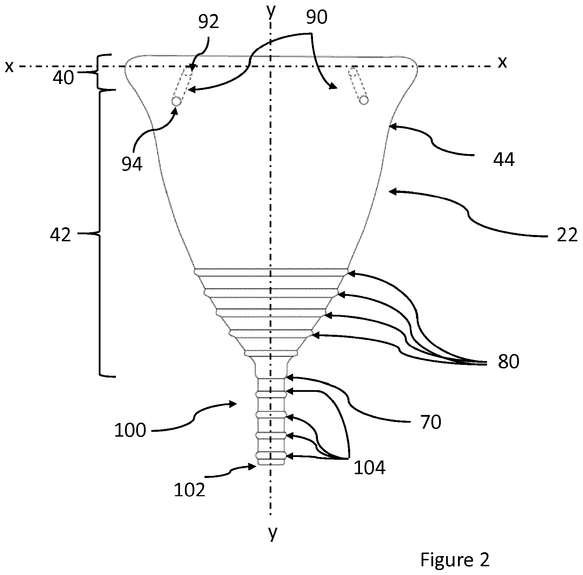

Preferably, the receptacle further comprises one or more holes 90 (shown in Figure 2) extending from the inner wall surface to the outer wall surface within the upper rim portion of the wall. The holes are preferably radially spaced apart from each other. When the cup is substantially upright, as shown in Figure 2, the inner end 92 of each hole is positioned higher than the outer end 94 of the hole relative to the longitudinal axis. The holes permit fluid communication between the inner wall surface and the outer wall surface such that as the cup is inserted into the vagina and removed therefrom, air pressure within the cavity is equalised with atmospheric pressure. The inclined angle of the holes limits the chance that fluid that has accumulated within the cavity leaks out through the holes, either when the cup is in situ or as it is removed. Preferably, the cup has between 2 and 8 holes, preferably between 3 and 5 holes or 4 holes. Preferably, the holes have a diameter of between 0.5 mm and 2.5 mm or 1 mm and 2 mm.

The receptacle further comprises grips 80 provided on the outer surface of the receptacle adjacent the base. The grips 80 shown in Figure 2 are in the form of concentric circles, however it will be appreciated that the grips may take other forms, for example, the grips may be in the form of concentric zigzags, curves, parallel vertical or horizontal lines or a series of concentric dots. The grips are preferably provided from the external base of the receptacle up to a distance that is 40%, 35%, 30% or 25% of the receptacle height. In an embodiment, the receptacle is provided with 2, 3, 4, 5 or 6 grips, spaced between 1 mm and 5mm, 2 mm and 4 mm or 3 mm and 3.5 mm apart and protruding a thickness of between 0.5 mm and 1.25 mm, 0.5 mm and 1.1 mm from the outer surface of the receptacle (measured in a direction orthogonal to a tangent of the outer surface). Without being bound by theory, it has been found that placement of the grips can have a serious impact on both ease of use and comfort when in situ. Grips are preferred by consumers to enable easy removal of the device when in situ, however, if grips are position too high on the outer surface of the receptacle wall or if they protrude too far from the receptacle outer surface, they may cause unnecessary discomfort to the user.

### Stem

A stem 12 is provided to aid extraction of the cup once in situ. The stem extends from the base of the receptacle and is substantially co-axial with the central axis of the receptacle. As shown in Figures 2 and 3B, the stem is generally elongate relative to the receptacle and has a substantially rectangular cross-section with a rounded base 102. The stem may have a uniform cross-section along its length or it may vary in thickness. Preferably, the stem has a cross-sectional area of between 5.5 mm² and 7.5 mm², 5.75 mm² and 7.25 mm², 6 mm² and 7 mm², 6.25 mm² and 6.75 mm² or 6.5 mm². The stem further has a length Lₛ measured from the external base of the receptacle to the base of the stem of between 13 mm and 32 mm, 15 mm and 30 mm, 17.5 mm and 27.5 mm, 20 mm and 25 mm. The stem should be sufficiently long to enable a user to firmly grasp it for removal of the cup, however, the stem should not be so long as to cause discomfort to the user when in situ. To enable cups having overall same or similar lengths, the size of the stem may be varied. For example, in an array having a normal sized cup (the first cup) and a larger cup (the second cup), the first cup may have a longer stem than the second cup. For example, the first cup may have a stem of between 18 mm and 20 mm in length, whereas the second cup may have a stem of between 12 mm and 14 mm in length.

The stem may be hollow or solid. Preferably, the stem is solid such that it can easily be snapped at different points by the user, thus providing flexibility for the user to choose a length they find practical and comfortable. The stem is further provided with grips 104 in the form of concentric rings. It will be appreciated that the grips may take other forms, for example, zig zags or vertical struts and that any number of grips may be provided. Preferably, the stem has between 3 and 7 grips, spaced between 1mm to 4 mm or 2 mm to 3 mm apart and protruding between 0.5 mm and 1.2 mm or 0.6 mm and 1.0 mm from the body of the stem.

The total length of the menstrual cup, including the stem and receptacle measured from the top open end (thus including rim) to the bottom of the stem 102 preferably does not exceed 70mm, 69 mm, 68 mm, 67 mm, 66 mm or 65 mm.

The above dimensions are all chosen to balance the tension between the outward size and comfort of the cup, the flexibility of the cup and finally, the capacity of the cup. The capacity of the cup varies when in use and can be measured in "resting" position, i.e., when in situ in a user's vagina or when outside of the body or in "compressed" position, i.e., during insertion or removal from the user's vagina.

### Array of Cups

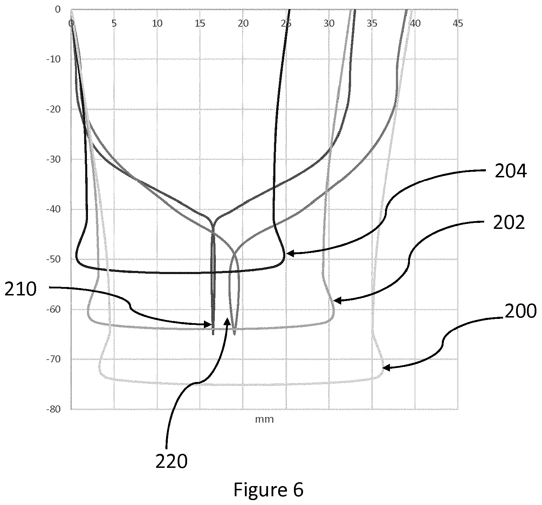

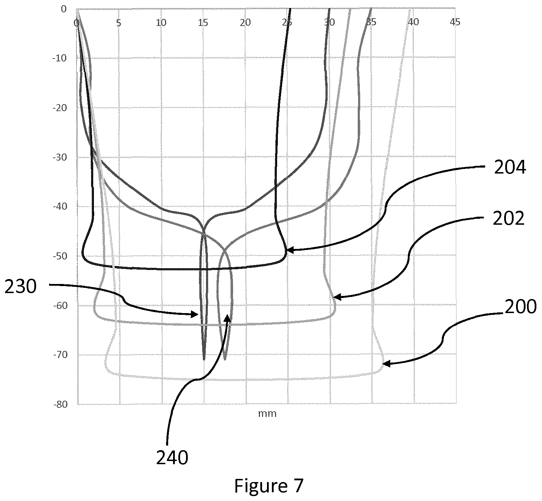

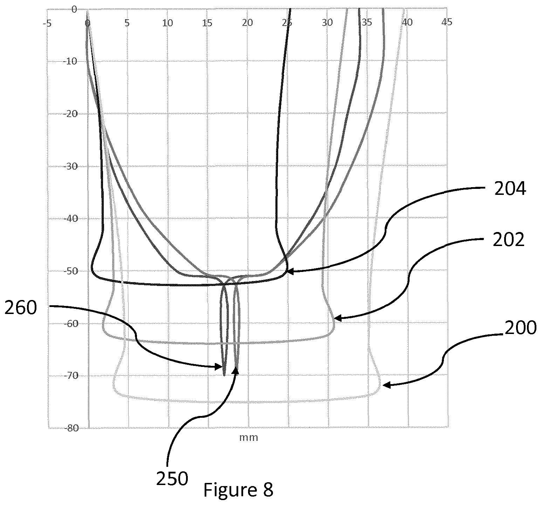

As described above, different cups may be provided having different dimensions to meet the needs of different users. The needs may be based on body size/dimensions or blood flow during menstruation. To date, there are no "one size fits all" cups available and, as a result, most commercially available cups are only suitable for a very small percentage of the female population. Some manufacturers have started making cups in two different sizes - typically a "regular" and "large". However, the present inventors have found that cups currently available on the market still do not meet the needs of most consumers and are typically still considered to be uncomfortable by many females. The dimensions of the leading menstrual cups in the market are shown in Tables I (regular size) and II (large size) below and Figures 6 and 7 show how this relates to average vagina sizes. Average vagina width at the cervical opening is 32.5, with a standard deviation of 7.13, however, width varies with age. Because the ruggae of the vagina can be stretched to accommodate a range of widths, larger width cups will nearly always offer a better stay-in-place experience than smaller cups. However, larger cups are also more likely to put pressure on the bladder or rectum. The benefit of the "V" shaped cup described herein is also illustrated in Figure 8, where it can be seen that the present cup(s) meet the needs of consumers by having a wide and thick rim but avoid pressing on the bladder or rectum. Without being bound by theory, it is thought that the cups described herein are suitable for use for approximately the 86% percentile of women and, based on conservative estimates, should be useful for between 11 hours to 19 hours of fluid flow (compared with an average tampon user).

**TABLE I**

| **Regular** | Comparative Cup 1 | Comparative Cup 2 | Inventive Cup (N) |
|---|---|---|---|
| L_{c} (mm) | 70 | 71 | 65 |
| Dₒ (mm) | 44 | 41 | 47 |
| Dᵢ (mm) | 34 | 30 | 38 |
| D₂₀ (mm) | - | - | 26 |
| Capacity (mL) | 27 | 23 | 24 |
| H_{c} (mm) | 53 | 45 | 44 |
| T_{w} (mm) | 3 | 2.5 | 3 |
| Tᵣ (mm) | 3.5 | 2.5 | 4.5 |

**TABLE II**

| **Large** | Comparative Cup 1 | Comparative Cup 2 | Inventive Cup (L) |
|---|---|---|---|
| L_{c} (mm) | 70 | 71 | 65 |
| Dₒ (mm) | 48 | 45 | 53 |
| Dᵢ (mm) | 37 | 35 | 44 |
| D₂₀ (mm) | - | - | 32 |
| Capacity (mL) | 33 | 35 | 37 |
| H_{c} (mm) | 53 | 49 | 49 |
| T_{w} (mm) | 3 | 2.5 | 3 |
| Tᵣ (mm) | 3.5 | 2.5 | 4.5, |

Figures 6, 7 and 8 show different vagina sizes as follows: average 200, 25^{th} percentile 202 and 5^{th} percentile 204. Comparative Cup 1 Regular 210 and Large 220 are shown in Figure 6, Comparative Cup 2 Regular 230 and Large 240 are shown in Figure 7, Inventive Cup Regular 250 and Large 260 are shown in Figure 8.

The above described "normal" and "large" menstrual cups may be displayed as an "array" of packages comprising menstrual cups, or other like-branded products, having different sizes as described above. While two different sizes of menstrual cups are described herein, it will be appreciated that such an array of menstrual cups may comprise more than two different sizes or even styles or materials of cups. Said packages may have the same brand and/or sub-brand and be oriented in proximity to each other within a given retail store (or advertised online together).

An "array of feminine articles" as used herein means a group of articles that are meant to provide a range of benefits to female consumer, specifically regarding menstrual and/or incontinence products. For example, a first plurality of packages may comprise products that are absorbent or non-absorbent and may be marketed for protection against menses leaking onto garments. A second plurality of packages may comprise products that are absorbent or non-absorbent and may be marketed for protection against urinary incontinence. And, each of the first plurality and second plurality of products are made by, made for, distributed by, and/or distributed for, a consumer article manufacturer. At least some of the first plurality of products may be used external to the body, e.g. pads which are attached to a user's undergarment. And, at least some of the first plurality of products may be intravaginal, e.g. tampons. Similarly, at least some of the second plurality of products may be used external to the body, e.g. pads which are attached to a user's undergarment. And, at least some of the second plurality of products may be intravaginal, e.g. pessaries.

Additionally, an array of feminine articles may comprise sub-arrays of specific products therein. For example, arrays of feminine articles may comprise a sub-array of menstrual cups (as described herein), sub-arrays of tampons having varying absorbent capacities and/or application methods, sub-arrays of feminine pads having varying sizes and absorbency levels, and/or a sub-arrays of incontinence products comprising incontinence pads (with varying sizes), incontinence pants (with varying sizes) and/or pessaries.

When compared to a sub-array of menstrual cups, a sub-array of tampons or a sub-array of pads (menstrual or incontinence) may offer more size choices than the sub-array of menstrual cups. For example, two or three different sizes of menstrual cups from which to choose may be provided for the consumer. In contrast, at least three different sizes of tampons may be provided from which to choose for the consumer. In some cases, four different sizes may be provided. Pads - whether menstrual or incontinence - may also provide for at least three different size options, at least four different size options, or at least five different size options from which to choose for the consumer.

Marketed for use means that on packaging in which products are situated, there is communication to the consumer regarding the intended use of the article. For example, for pessaries, packaging may include statements such as "for urinary incontinence" or "protection against bladder leaks." For menstrual products, packaging may include statements referring to periods and/or menstrual cycles in general.

Arrays can have the same brand identifier or brand identifiers may be different among different product forms or different intended use, e.g. pads versus intravaginal or menstrual products versus incontinence products. Brand identifiers of consumer products can include brand names, e.g. "Tampax", and/or may include like packaging elements (e.g., packaging material type, film, paper, dominant colour, design theme, etc.) that conveys to consumers that the different individual packages are part of a larger line-up. The packages may feature the same overall look but use different colour combinations to indicate differences (e.g., capacity or functionality) to users.

In addition to brand names, arrays may also utilize the same sub-brand, for example "Tampax Pearl". Other products within the array may have the same brand identifier "Tampax" but may utilize a different sub-brand, e.g. "Tampax Naturals". Still in other examples, the sub-brand of one product may be utilized as the brand identifier of a second product within the array. Arrays also often have the same trademarks, including trademarks of the brand name, sub-brand, and/or features and/or benefits across the line-up. "On-line Array" means an "Array" distributed by a common on-line source. In yet another example, products within the array may share the same brand identifier but may be marketed for differing uses. As an example, "Always Ultra Thin" is marketed for menstrual use, and "Always Discreet" is marketed for incontinence use.

Arrays of the present description may comprise a first package comprising a first product and a second package comprising a second product. The first product and the second product are made by, made for, distributed by, or distributed for a manufacturer of consumer articles. The first and the second products may comprise the same brand identifier. The first product and the second product may be absorbent or non-absorbent. The first product and the second product may be utilized externally or may be intravaginal. Examples of intravaginal products suitable for menstrual use include tampons and cups as described herein. An example of intravaginal products suitable for incontinence use include pessaries. Examples of externally used products suitable for menstrual use include feminine hygiene pads and wipes. Examples of externally used products suitable for incontinence use include incontinence pads or pants and wipes.

With the above in mind, the first product may be a cup or a plurality of cups as described herein. The second product may be a pessary. Or, the second product may comprise an incontinence pad or pant. Where the second product is marketed for menstrual use, the second product may comprise a tampon or may comprise a feminine hygiene pad.

Some examples of non-absorbent products include the cups described herein and pessary devices. Pessaries are utilized for feminine incontinence and are currently available on the market. In general pessaries are marketed for incontinence and lack the ability to absorb substantial amounts of fluid unlike their tampon and pad counterparts. Additionally, generally pessaries lack the ability to store fluid unlike their cup counterparts. So, pessaries are generally considered to be non-absorbent and non-storage devices. Some examples of suitable pessaries and methods of making are discussed in U.S. Patent Nos. 8,926,493; 9,078,726; 9,320,640; 9,744,630; 8,919,345; 9,433,523; 9,339,364; 9,439,748; in U.S. Patent Application Publication Nos. 2012/0259160; 2012/0259162; 2012/0259164; 2012/0259166; 2012/0259167; 2016/0235583; 2014/0216466; 2015/0359684; 2015/0351975; and 2016/0374788.

In one specific example, an array of menstrual products may comprise one or more menstrual cups that share the same brand identifier and one or more disposable absorbent products, for example: tampons, sanitary or incontinence pads, incontinence pants (diapers) incontinence devices, e.g. pessaries, and/or wipes that share the same brand identifier. Alternatively, the brand identifier of complimentary products may be different.

Regardless of whether brand identifiers of the products are the same or different, the first product, the second product, and optionally additional products, may be sold together as a kit of products. For example, such a kit may comprise one or more menstrual cups of different sizes with the brand name "Tampax" and liners for use with the menstrual cups with a brand name "Always" or wet wipes with the same or another brand name. Such kits will likely be packaged together to clearly indicate to consumers that they should be used in conjunction with one another.

In other examples, an array may comprise a first non-absorbent product marketed for use with menstrual fluid, e.g. a cup, and a second non-absorbent and non-storage product marketed for use with urinary incontinence e.g. a pessary. The first non-absorbent product and the second non-absorbent, non-storage product may be marketed under the same brand identifier.

### Materials

Cups as described herein may be formed of an elastomeric material, such as an organosilicone oxide polymer, i.e., a silicone rubber. Silicone rubber is preferred because it rarely (if ever) causes skin irritation, and it has the necessary resiliency and durability. Furthermore, silicone rubber may be placed in boiling water for sterilization without damaging the article. Preferably, the silicone rubber is of medical grade.

The receptacle and stem are preferably formed integrally and of the same material. Although it will be appreciated that it would be possible to form the parts separately and then to attach them by known means.

The cup may be formed by an injection molding process. However, it will be appreciated that other known methods of forming articles from plastics and other similar materials may be used. Where the cup is formed by a molding process, preferably the holes are introduced during the molding process (as compared with boring holes through the rim wall after molding). By forming the holes as part of the molding process, this increases the ease of cleaning the cup.

### Compression of Cup

The dimensions of rim and wall thickness and the materials making up the cup influence how the cup behaves upon entry and exit of a user's vagina and while in situ. The present inventors have found that the dimensions and materials chosen above provide optimal conditions for this and, in particular, enable the cup to stay in place while being comfortable before, during and after use. The compression test method below measures the force required to flex the cup (as described below). Preferably, a cup as described herein requires a compression force of between 1N - 5N, 1.5N to 4.5N or 2.0N to 4.0N at the top (as defined below) of the cup, a compression force of between 4.0N to 8.5N, 4.5N to 8.0N or 5.0N to 7.5N at the middle of the cup and a compression force of between 4.5N to 13.5N, 5.0N to 12.0N, 5.5N to 11:0N, 6.0N to 10.0N, 6.5N to 9.0N or 7.0N to 8.0N at the bottom.

The following results are the average forces measured using the compression test method on a series of 210 cups having the dimensions of Inventive Cups N and L, shown in Tables II and III:

**Table III:**

| | | Mean Load (N) | Range (N) |
|---|---|---|---|
| Inventive Cup (N) | Top | 3.17 | 2.7-3.8 |
| | Middle | 6.49 | 5.3-7.6 |
| | Bottom | 7.97 | 5.8-7.1 |
| Inventive Cup (L) | Top | 2.65 | 2.2-3 |
| | Middle | 5.79 | 5-6.6 |
| | Bottom | 7.44 | 2.8-12.1 |

### Compression Test for Menstrual Cup

The maximum compression strength at three different location on a menstrual cup is measured on a constant rate of extension tensile tester with compression load cell capability. A suitable instrument is the MTS Alliance using Testworks 4.0 Software, as available from MTS Systems Corp., Eden Prairie, MN, or equivalent, equipped with a load cell for which the forces measured are within 10% to 90% of the limit of the cell. All testing is performed in a room controlled at 23°C ± 2 C° and 50% ± 2% relative humidity and test samples are conditioned in this environment for at least 2 hours prior to testing.

The bottom stationary fixture consists of a rigid, horizontal platen large enough to support the sample holder, described herein. The upper fixture is a cylindrical plunger having an overall length of about 30 mm with a diameter of 6.0 mm. The contact tip is a ball nose having a radius of 3.0 mm. The plunger has an adapter compatible with the mount on the load cell capable of securing the plunger orthogonal to the bottom platen.

The sample holder is constructed in such a way that the cup is supported with its radial axis (depicted as y in Figure 2) oriented parallel to the bottom horizontal plate. The sample holder must be rigid, nondeformable and the interior must conform to the shape of the cup's surface. The holder is deep enough to allow one half of the cup (divided along the radial axis, depicted as y) to rest securely inside the holder and it enables testing of the cup at different locations parallel to its radial axis. One suitable way to make said sample holder is to create a 3D-printed plastic mold. This type of design ensures the test sample remains in a fixed position during testing while also allowing the re-positioning necessary to analyze multiple locations.

For the purpose of this test, the longitudinal end of the test sample that is open to collect fluid and may contain a rim will be known as the "top" (region **40** in Figure 2). The longitudinal end of the test sample that is at the base of the receptacle (excluding any stem present) will be known as the "bottom" (region **70** in Figure 2).

Obtain five substantially similar test samples. Mark the test locations (measured parallel to the radial axis) on the outer surface of the test sample as follows. The Bottom Location is 5.0 mm from the bottom of the cup receptacle. The Mid Location is above the bottom of the cup receptacle at a distance that is equal to 30.0% of the distance between the top and bottom of the cup receptacle determined along the radial axis. The Top Location is 5.0 mm from the top edge of the cup receptacle.

Program the tensile tester as a compression test to lower the crosshead at 2.0 mm per second. The ball-nose plunger moves down until the desired displacement is reached. Displacements for each test location are as follows. Bottom Location is 3.0 mm, Mid Location is 5.0 mm and Top Location is 10.0 mm. Force (N) and displacement (mm) are recorded at a data acquisition rate of 50 Hz. Insert the test sample into its respective holder and place it onto the bottom platen of the tensile tester. Align the pre-marked test location under the center of the ball nose of the plunger. At low speed, lower the crosshead until it exerts a negligible force (< 0.03 N) onto the test sample at the test location, then zero the crosshead. Start the test, ensuring the correct displacement is used for the respective test location. Record the test location and the Maximum Peak Force to the nearest 0.01 N.

Repeat in like fashion for all 3 test locations on all 5 replicates. Calculate the arithmetic mean for Maximum Peak Force to the nearest 0.01 N for each test location (Bottom, Mid, Top) and report as Compression Strength for each test location.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A kit comprising an array of feminine articles, the array comprising:
a first package comprising a menstrual cup (10) having a first size and capacity marketed for menstrual use;
a second package comprising a second product marketed for menstrual use, wherein the second product comprises a feminine hygiene pad;
wherein the menstrual cup has a rim (40) extending around the circumference of the top end and having an inner diameter Dᵢ across the top end of more than 35mm and a thickness Tᵣ of between 3 mm and 5 mm; and a length of less than 53 mm from the top end to the base of the receptacle (20), and wherein the menstrual cup and the second product are made by, made for, distributed by, and/or distributed for a manufacturer of feminine consumer articles.

2. . The kit of claim 1, further comprising a third package comprising a wipe, wherein the third package and the first package comprise the same brand identifier.

3. . The kit of any of the preceding claims, wherein the first package and the second package comprise the same brand identifier and/or the same brand name.

## Patentansprüche

1. Kit, umfassend eine Anordnung von Damenartikeln, die Anordnung umfassend:
eine erste Verpackung, umfassend eine Menstruationstasse (10), die eine erste Größe und Kapazität aufweist, die für eine menstruationsbezogene Verwendung vermarktet wird;
eine zweite Verpackung, umfassend ein zweites Produkt, das für die menstruationsbezogene Verwendung vermarktet wird, wobei das zweite Produkt eine Damenhygieneeinlage umfasst;
wobei die Menstruationstasse einen Rand (40) aufweist, der sich um den Umfang des oberen Endes herum erstreckt und einen Innendurchmesser Dᵢ über das obere Ende hinweg von mehr als 35 mm und eine Dicke Tᵣ zwischen 3 mm und 5 mm aufweist; und eine Länge von weniger als 53 mm von dem oberen Ende zu der Basis des Behälters (20), und wobei die Menstruationstasse und das zweite Produkt durch einen Hersteller von Damenverbrauchsartikeln hergestellt, für diesen hergestellt, durch diesen vertrieben und/oder für diesen vertrieben werden.

2. Kit nach Anspruch 1, ferner umfassend eine dritte Verpackung, umfassend ein Wischtuch, wobei die dritte Verpackung und die erste Verpackung die gleiche Markenkennung umfassen.

3. Kit nach einem der vorstehenden Ansprüche, wobei die erste Verpackung und die zweite Verpackung die gleiche Markenkennung und/oder den gleichen Markennamen umfassen.

## Revendications

1. Kit comprenant un ensemble d'articles féminins, l'ensemble comprenant :
un premier emballage comprenant une coupe menstruelle (10) ayant une première taille et une première capacité, commercialisée pour un usage menstruel ;
un deuxième emballage comprenant un second produit commercialisé pour un usage menstruel, dans lequel le second produit comprend une serviette hygiénique féminine ;
dans lequel la coupe menstruelle a un rebord (40) s'étendant autour de la circonférence de l'extrémité supérieure et ayant un diamètre interne Dᵢ sur l'ensemble de l'extrémité supérieure de plus de 35 mm et une épaisseur Tᵣ comprise entre 3 mm et 5 mm ; et une longueur inférieure à 53 mm de l'extrémité supérieure à la base du réceptacle (20), et dans lequel la coupe menstruelle et le second produit sont fabriqués par, fabriqués pour, distribués par et/ou distribués pour un fabricant d'articles de consommation féminine.

2. Kit selon la revendication 1, comprenant en outre un troisième emballage comprenant une lingette, dans lequel le troisième emballage et le premier emballage comprennent le même identifiant de marque.

3. Kit selon l'une quelconque des revendications précédentes, dans lequel les premier et deuxième emballages comprennent le même identifiant de marque et/ou le même nom de sous-marque.
